**Europäisches Patentamt**

(19) **European Patent Office**

**Office européen des brevets**

(11) Veröffentlichungsnummer: **0 176 692**
**B1**

(12) **EUROPÄISCHE PATENTSCHRIFT**

(45) Veröffentlichungstag der Patentschrift:
**01.06.88**

(21) Anmeldenummer: **85109614.9**

(22) Anmeldetag: **31.07.85**

(51) Int. Cl.⁴: **C 04 B 35/52, H 01 G 9/04,**
**C 01 B 31/02**

(54) Verfahren zur Herstellung von Glaskohlenstoff.

(30) Priorität: **13.08.84 DE 3429794**

(43) Veröffentlichungstag der Anmeldung:
**09.04.86 Patentblatt 86/15**

(45) Bekanntmachung des Hinweises auf die Patenterteilung:
**01.06.88 Patentblatt 88/22**

(84) Benannte Vertragsstaaten:
**DE FR GB IT NL SE**

(56) Entgegenhaltungen:
**EP - A - 0 036 601**
**DE - A - 1 694 909**
**DE - A - 2 418 507**
**FR - A - 2 380 985**

**CHEMICAL ABSTRACTS, Band 99, Nr. 26, Dezember 1983, Seite 142, no. 214898s, Columbus, Ohio, US; & FI - A - 64 117 (AALTOSEN TEHTAAT OY) 30-06-1983**

(73) Patentinhaber: **Siemens Aktiengesellschaft Berlin und München, Wittelsbacherplatz 2, D-8000 München 2 (DE)**

(72) Erfinder: **Edeling, Martin, Dr., Barkhovenallee 22, D-4330 Essen 16 (DE)**
Erfinder: **Gebhardt, Ulrich, Zedernstrasse 18, D-8521 Langensendelbach (DE)**

ACTORUM AG

## Beschreibung

Die Erfindung betrifft ein Verfahren zur Herstellung von Glaskohlenstoff durch Härtung und Pyrolyse von duroplastischen carbonisierbaren Harzen sowie die Verwendung des nach diesem Verfahren hergestellten Glaskohlenstoffs.

Kondensatoren mit grosser Volumenkapazität lassen sich unter Ausnützung der elektrochemischen Doppelschicht verwirklichen. Für derartige elektrochemische Doppelschichtkondensatoren werden poröse Elektroden mit einer grossen inneren Oberfläche benötigt, die mit einem Elektrolyten hoher Leitfähigkeit in Kontakt gebracht werden. Um einen mehrzelligen Aufbau und damit grössere Nennspannungen als die Zersetzungsspannung des Elektrolyten realisieren zu können, müssen dabei die einzelnen Elektrolyträume voneinander getrennt werden.

Die bei den Elektroden erforderliche grosse innere Oberfläche kann beispielsweise durch Verwendung von gebundener Aktivkohle (DE-A 3 000 777 und DE-A 3 046 252) oder aktiviertem Glaskohlenstoff (DE-A 3 011 701) erreicht werden. Dabei bietet Glaskohlenstoff gute elektrische und mechanische Eigenschaften, eine problemlose Formgebung und die erforderliche Gasdichtigkeit, durch die Aktivierung können aber nur relativ dünne Aktivschichten mit einer Dicke von 10 bis 50 μm erzeugt werden und damit lediglich Kapazitäten von einigen 100 mF · cm$^{-2}$. Dagegen lassen sich mit gebundener Aktivkohle, aufgrund der hohen Porosität, Doppelschichtkapazitäten von bis zu 10 F · cm$^{-2}$ erzielen (bei Elektrodenstärken von etwa 1 mm), die Elektroden müssen dabei jedoch mechanisch gestützt und zusätzlich abgedichtet werden; ausserdem können wegen der verwendeten Bindemittel nur mässige elektronische Leitfähigkeiten realisiert werden.

Aufgabe der Erfindung ist es, das Verfahren der eingangs genannten Art zur Herstellung von Glaskohlenstoff aus duroplastischen carbonisierbaren Harzen derart auszugestalten, dass Glaskohlenstoff hergestellt werden kann, der neben den guten elektrischen und mechanischen Eigenschaften gleichzeitig eine hohe Doppelschichtkapazität aufweist.

Dies wird erfindungsgemäss dadurch erreicht, dass zur Herstellung von porösem Glaskohlenstoff dem Harz 1 bis 10 Masse-% eines im Harz löslichen, rückstandsfrei sublimierbaren Salzes einer Fettsäure mit 10 bis 20 Kohlenstoffatomen zugesetzt werden.

Der nach dem erfindungsgemässen Verfahren hergestellte Glaskohlenstoff verbindet die guten elektrischen und mechanischen Eigenschaften des Glaskohlenstoffs mit der hohen Doppelschichtkapazität von Aktivkohle, er weist nämlich ebenso wie diese eine hohe Porosität auf. Dies liegt darin begründet, dass die dem Harz zugesetzten Verbindungen als Porenbildner fungieren und eine Makroporosität bewirken. Die im Glaskohlenstoff aufgrund der Salz-Zusätze zum Harz gebildeten (Makro-)Poren weisen dabei einen Durchmesser im μm-Bereich (10$^{-6}$ m) auf.

Aufgrund seiner guten Eigenschaften eignet sich der nach dem erfindungsgemässen Verfahren hergestellte makroporöse Glaskohlenstoff in hervorragender Weise als Elektrodenmaterial in elektrochemischen Doppelschichtkondensatoren. Elektroden aus einem derartigen Material sind nämlich nicht nur mechanisch stabil und gasdicht, sondern weisen auch eine gute elektrische Leitfähigkeit und eine hohe Doppelschichtkapazität auf. Kondensatoren mit derartigen Elektroden eignen sich deshalb in besonderer Weise zur Notstromversorgung für integrierte Halbleiterspeicher bei Netzunterbrechungen.

Die Herstellung von makroporösem Glaskohlenstoff ist an sich bereits bekannt (GP-A 1 031 126 und US-A 3 446 593). Dabei wird ein makroporöses Trägermaterial, wie Polyurethanschaum, mit einem duroplastischem Harz getränkt und das imprägnierte Trägermaterial nachfolgend carbonisiert; dabei entsteht hochporöser Glaskohlenstoff. Ein derartiges Material eignet sich aber nicht zur Herstellung von Elektroden mit hoher Doppelschichtkapazität, da es dafür zu porös ist (schwammartige Struktur); darüber hinaus sind die «Makroporen» so gross (> ca. 0,1 mm), dass das Material in hohem Masse gas- und flüssigkeitsdurchlässig ist.

Bei dem aus der FR-A 2 380 985 bekannten Verfahren zur Herstellung von Glaskohlenstoff durch Härtung und Pyrolyse von duroplastischen carbonisierbaren Harzen wird von lösungsmittelhaltigen Mischungen ausgegangen. Durch das Austreten der Lösungsmittel aus der Polymermatrix entstehen Poren, die sich bei höherer Temperatur zusammenziehen.

Beim erfindungsgemässen Verfahren wird als carbonisierbares Harz vorzugsweise Furfurylalkohol oder ein Phenol-Furanharz eingesetzt. Es können aber auch andere duroplastische carbonisierbare Harze verwendet werden, beispielsweise Phenol-Formaldehydharze.

Dem duroplastischen carbonisierbaren Harz wird erfindungsgemäss – vor der Härtung und der Pyrolyse – ein Salz einer Fettsäure zugesetzt; dabei können auch Gemische derartiger Verbindungen zum Einsatz gelangen. Mit «Fettsäuren» werden aliphatische Carbonsäuren der allgemeinen Formel RCOOH bezeichnet, und zwar sowohl gesättigte als auch ungesättigte Verbindungen. Beim erfindungsgemässen Verfahren werden dabei Derivate von Fettsäuren mit 10 bis 20 Kohlenstoffatomen eingesetzt d.h. von sogenannten mittleren bzw. höheren Fettsäuren.

Die Fettsäurederivate, die den Harzen in einer Menge von 1 bis 10 Masse-%, vorzugsweise 4 bis 5 Masse-%, zugesetzt werden, sind Salze der Fettsäuren, d.h. Verbindungen der allgemeinen Formel RCOOMe (Me = Metall). Bevorzugt werden dabei Zinkstearat (C$_{17}$H$_{35}$COOZn) und Zinkundecylenat (C$_{10}$H$_{19}$COOZn) verwendet; Stearinsäure ist eine gesättigte Fettsäure, Undecylensäure (bzw. 10-Undecensäure) eine einfach ungesättigte Fettsäure. Weitere einsetzbare Salze sind beispielsweise Magnesiumstearat und Salze

der Palmitinsäure ($C_{15}H_{31}COOH$). Die verwendeten Salze müssen im Harz löslich sein. Darunter ist zu verstehen, dass sie sich entweder bereits in den Ausgangssubstanzen lösen oder aber während der Polymerisation des Harzes.

Die Härtung von duroplastischen Harzen bzw. Kunststoffen kann mit basischen oder sauren Verbindungen erfolgen. Beim erfindungsgemässen Verfahren werden als Härter im allgemeinen starke Säuren eingesetzt, vorzugsweise wird p-Toluolsulfonsäure verwendet. Die Härtung selbst erfolgt bevorzugt bei Temperaturen von 150 bis 200°C; Dauer der Härtung: etwa 1 bis 5 h.

Bei der Herstellung von Elektroden wird im allgemeinen in folgender Weise verfahren. Das Harz wird zunächst polymerisiert, und zwar bei Temperaturen bis etwa 100°C. Aus diesem Harzpolymerisat, das vorteilhaft in Form einer flexiblen Folie vorliegt, werden dann Elektroden-Rohlinge in der gewünschten Form hergestellt, beispielsweise durch Stanzen; dabei ist die lineare Schrumpfung des Polymerisats von ca. 20% während der Pyrolyse zu berücksichtigen. Die Elektroden-Rohlinge werden nachfolgend gehärtet. Nach der Härtung können sie, soweit erforderlich, noch mechanisch bearbeitet werden. Anschliessend werden die Rohlinge dann der Pyrolyse unterworfen.

Die Pyrolyse erfolgt vorzugsweise bei Temperaturen bis zu 1100°C. Bei der Pyrolyse wird der duroplastische Kunststoff carbonisiert und dabei in Glaskohlenstoff übergeführt. Gleichzeitig sublimiert der Porenbildner, d.h. das Salz der Fettsäure, ab, wobei im Glaskohlenstoff eine makroporöse Struktur gebildet wird. Die Pyrolysedauer beträgt im allgemeinen etwa 12 bis 36 h (einschliesslich Aufheiz- und Abkühlzeit), vorzugweise ca. 24 h. Die Endtemperatur wird dabei für etwa 1 bis 5 h gehalten.

Der durch die Pyrolyse erhaltene Glaskohlenstoff kann vorteilhaft noch aktiviert werden, insbesondere dann, wenn er als Elektrodenmaterial Verwendung finden soll. Dabei wird auf dem Glaskohlenstoff oberflächlich eine mikroporöse Struktur mit Poren im nm-Bereich ($10^{-9}$ m und darunter) erzeugt. Durch die Aktivierung, die vorzugsweise durch Tempern an der Luft bei Temperaturen zwischen 450 und 500°C erfolgt (Dauer: ca. 3 bis 5 h), kann die Doppelschichtkapazität des Glaskohlenstoffs bzw. der Elektroden noch weiter erhöht werden.

Nach dem erfindungsgemässen Verfahren hergestellter und aktivierter Glaskohlenstoff weist – beispielsweise bei einer Schichtdicke von 1 mm – eine Doppelschichtkapazität von über 10 F·$cm^{-2}$ auf (in 3,6 M $H_2SO_4$). Die Kapazität ist dabei, ebenso wie bei Aktivkohle, frequenzabhängig, wobei die volle Kapazität bei Frequenzen unterhalb 1 bis 10 mHz zugänglich ist. Der spezifische elektronische Widerstand des porösen Glaskohlenstoffs liegt unter 10 mΩ·cm.

Neben der Verwendung als Elektrodenmaterial für elektrochemische Doppelschichtkondensatoren kann der nach dem erfindungsgemässen Verfahren hergestellte Glaskohlenstoff beispielsweise auch zur Herstellung von Elektroden dienen, die in der Elektromedizin Verwendung finden. Dies sind vor allem Reizelektroden, insbesondere für Herzschrittmacher (siehe dazu DE-A 2 613 072).

Anhand von Ausführungsbeispielen und einer Figur soll die Erfindung noch näher erläutert werden.

Zur Herstellung makroporöser Glaskohlenstoffelektroden mit hoher Doppelschichtkapazität wird beispielsweise folgendermassen verfahren. In 150 ml Furfurylalkohol werden 7,5 g Zinkstearat (ca. 4,4 Masse-%) eingerührt und anschliessend werden 15 ml einer 10%igen Lösung von p-Toluolsulfonsäure in Ethanol zugegeben. Das Gemisch wird 2 h bei Raumtemperatur gerührt, dann auf eine 80°C heisse Platte (30 cm × 30 cm) aus Polypropylen gegossen und über Nacht bei dieser Temperatur gehalten. Aus der dabei erhaltenen flexiblen, selbsttragenden Folie mit einer Dicke von ca. 1,5 mm werden Elektroden-Rohlinge mit den gewünschten Abmessungen ausgestanzt. Diese Rohlinge werden dann bei einer Temperatur von ca. 200°C gehärtet; Dauer: ca. 1 h.

Um eine hohe Gasdichtigkeit zu gewährleisten, können die Elektroden zusätzlich noch abgedichtet werden. Wenn dies lediglich auf einer Seite erfolgen soll, so wird folgendermassen verfahren. Die Rohlinge werden sandgestrahlt, einseitig mit Harz (20 ml Furfurylalkohol + 1 ml der obengenannten Härter-Lösung) bestrichen und unter einem IR-Strahler ausgehärtet; diese Prozedur kann auch wiederholt werden. Derart behandelte Elektroden weisen eine Helium-Leckrate von lediglich $10^{-7}$ mbar·$1$·$s^{-1}$·$cm^{-2}$ auf. Auf die genannte Weise ist es auch möglich, zwei Elektroden miteinander zu verkleben, um symmetrische, gasdichte Elektroden für eine bipolare Anordnung herzustellen.

Die gehärteten Elektroden-Rohlinge werden dann der Pyrolyse unterworfen. Dazu werden sie unter einer Inertgasatmosphäre, beispielsweise unter Argon, bis auf eine Temperatur von 1100°C erhitzt (Gesamtdauer der Pyrolyse: ca. 24 h). Während der Pyrolyse sublimiert das Zinkstearat ab und hinterlässt im gebildeten Glaskohlenstoff eine makroporöse Struktur, die im Rasterelektronenmikroskop deutlich zu erkennen ist.

Die fertigen Elektroden, die – bei einer Dicke von 1 mm – eine Elektrodenfläche von 4,9 $cm^2$ aufweisen, wurden mittels der Elektronenstrahl-Röntgenmikroanalyse auf Restgehalte an Zink untersucht. Dabei konnte, bei einer Nachweisgrenze unterhalb 0,1 Masse-%, in der Oberfläche und in den Poren kein Zink nachgewiesen werden. Der elektronische Widerstand der Glaskohlenstoffelektroden ergibt sich zu 4,5 mΩ·cm, er kann somit – bei einem Kondensator – gegenüber dem Elektrolytwiderstand vernachlässigt werden. Im übrigen ist der elektronische Widerstand abhängig von der Pyrolyse-Endtemperatur.

Zur Aktivierung wurden die Elektroden bei ca. 470°C etwa 3 h an der Luft getempert. Dann wurden in einer Halbzelle mit 3,6 M $H_2SO_4$ als Elektrolyt Kapazitätsmessungen durchgeführt. Die Doppelschichtkapazität kann dabei sowohl mittels Dreieckspannungsbelastung als auch durch Impedanzmessungen ermittelt werden, wobei zu berücksichtigen ist, dass die volle Kapazität erst bei kleinen Spannungsgeschwindigkeiten bzw. niedrigen Frequenzen zugänglich ist. Bei potentiodynamischen Messungen zwischen 100 und 300 mV vs $H_2$rev und einer Vorschubgeschwindigkeit von $10^{-4}$ V/s erhält man Kapazitäten von 12,5 F $\cdot$ cm$^{-2}$ (Elektrodendicke: 1 mm). Aus den Impedanzmessungen ergibt sich, dass – aufgrund der Porenstruktur – die Kapazität oberhalb 10 mHz absinkt. Bei 1 mHz beträgt die Kapazität 10,9 F $\cdot$ cm$^{-2}$; bezogen auf das Volumen ergibt sich eine Kapazität von 100 F $\cdot$ cm$^{-3}$.

Unter Verwendung von zwei in der vorstehend beschriebenen Weise hergestellten Glaskohlenstoffelektroden wurde ein einzelliger Modellkondensator gebaut; als Elektrolyt diente 3,6 M $H_2SO_4$. Zur Kontaktierung wurde auf jede Elektrodenrückseite eine Kupferplatte aufgeklebt und darauf ein Ableitkabel aufgelötet. An einem derartigen Kondensator wird eine Kapazität von 30 F gemessen, was einer Flächenkapazität von 12,2 F $\cdot$ cm$^{-2}$ entspricht.

Mit dem beschriebenen Modellkondensator wurden Lade- und Entladekurven aufgezeichnet. Wird der vollständig entladene Kondensator mit 1 V geladen, so fliesst nach einer Stunde noch ein Reststrom von 1,8 mA (bei einer Elektrodenfläche von 4,9 cm$^2$); nach 16 h fliesst noch ein Strom von 0,1 mA.

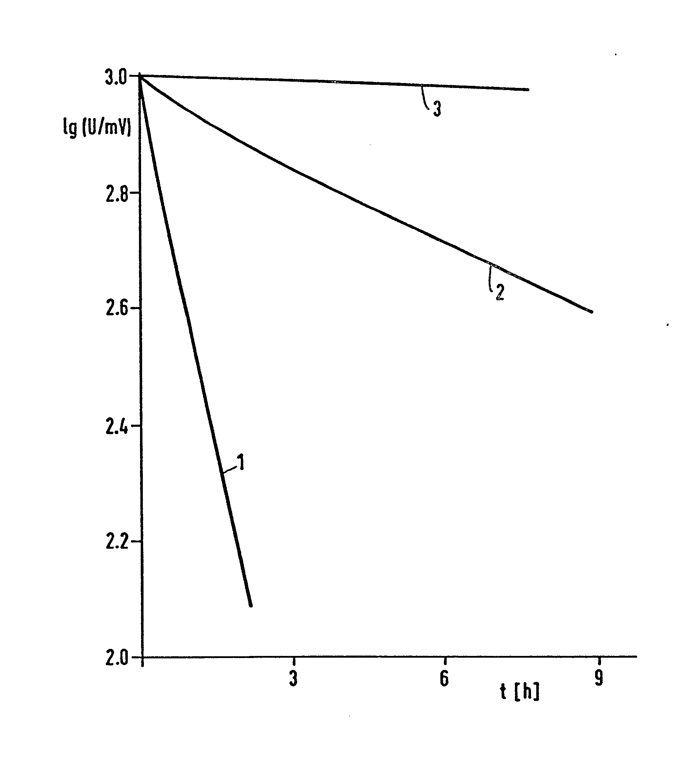

In der Figur sind Entladungskurven dargestellt, und zwar bei einem Lastwiderstand R von 100 Ω (Kurve 1), 1 kΩ (Kurve 2) und $10^4$ kΩ (Kurve 3), d.h. bei Selbstentladung. Der nahezu gerade Kurvenverlauf deutet auf einen idealen Kondensator hin. Aus der Selbstentladung errechnet sich ein Leckwiderstand von 15 kΩ und damit ergibt sich eine Kapazität von 37 F, was einer Flächenkapazität von ca. 15 F $\cdot$ cm$^{-2}$ entspricht.

Weitere makroporöse Glaskohlenstoffelektroden wurden in folgender Weise hergestellt:

a) 150 ml Furfurylalkohol werden mit 7,5 g Zinkundecylenat und 10 ml einer 20%igen Lösung von p-Toluolsulfonsäure in Ethanol versetzt und 2 h bei Raumtemperatur gerührt. Das Gemisch wird dann auf eine Unterlage aus Polypropylen ausgegossen und über Nacht bei einer Temperatur von 90°C poylmerisiert. Dabei wird eine flexible, selbsttragende Folie erhalten, die in der vorstehend beschriebenen Weise zu Elektroden weiterverarbeitet wird. Die nach der Pyrolyse und nachfolgender Aktivierung (5 h bei 470°C an der Luft) erhaltenen Elektroden weisen – bei einer Dicke von 0,8 mm – eine Doppelschichtkapazität von 2 F $\cdot$ cm$^{-2}$ bzw. 25 F $\cdot$ cm$^{-3}$ auf.

b) 20 g eines käuflichen Phenol-Furanharzes werden bei 50°C mit 1 g Zinkstearat versetzt und dann intensiv gerührt. Anschliessend wird 1 ml einer 20%igen Lösung von p-Toluolsulfonsäure in Ethanol zugegeben und bei 80°C in der beschriebenen Weise polymerisiert. Nach Härtung, Pyrolyse und Aktivierung (5 h bei 470°C) werden Elektroden erhalten, welche – bei einer Dicke von 1 mm – eine Doppelschichtkapazität von 3 F $\cdot$ cm$^{-2}$ aufweisen.

Das erfindungsgemässe Verfahren ist nicht auf die vorstehend erläuterten Ausführungsbeispiele beschränkt, es kann vielmehr in mancherlei Hinsicht variiert werden. So kann beispielsweise die Polymerisation des Harzes in zwei Stufen erfolgen. Dazu wird in der Weise vorgegangen, dass dem Harz zunächst, d.h. in einer ersten Stufe, nur ein Teil des insgesamt erforderlichen Härters zugesetzt wird. Hierbei resultiert dann ein Harz-Vorpolymerisat, das bei Raumtemperatur für einige Zeit, d.h. bis zu mehreren Tagen, lagerfähig ist und in einer zweiten Stufe – unter Zusatz des restlichen Härters – vollständig polymerisiert wird. Hieran schliesst sich dann, wie beim einstufigen Prozess, die Härtung an.

## Patentansprüche

1. Verfahren zur Herstellung von Glaskohlenstoff durch Härtung und Pyrolyse von duroplastischen carbonisierbaren Harzen, dadurch gekennzeichnet, dass zur Herstellung von porösem Glaskohlenstoff dem Harz 1 bis 10 Masse-% eines im Harz löslichen, rückstandsfrei sublimierbaren Salzes einer Fettsäure mit 10 bis 20 Kohlenstoffatomen zugesetzt werden.

2. Verfahren nach Anspruch 1, dadurch gekennzeichnet, dass als Harz Furfurylalkohol verwendet wird.

3. Verfahren nach Anspruch 1, dadurch gekennzeichnet, dass ein Phenol-Furanharz verwendet wird.

4. Verfahren nach einem der Ansprüche 1 bis 3, dadurch gekennzeichnet, dass dem Harz Zinkstearat zugesetzt wird.

5. Verfahren nach einem der Ansprüche 1 bis 3, dadurch gekennzeichnet, dass dem Harz Zinkundecylenat zugesetzt wird.

6. Verfahren nach einem oder mehreren der Ansprüche 1 bis 5, dadurch gekennzeichnet, dass zur Härtung des Harzes p-Toluolsulfonsäure verwendet wird.

7. Verfahren nach einem oder mehreren der Ansprüche 1 bis 6, dadurch gekennzeichnet, dass das Harz bei Temperaturen von 150 bis 200°C gehärtet wird.

8. Verfahren nach einem oder mehreren der Ansprüche 1 bis 7, dadurch gekennzeichnet, dass die Pyrolyse bei Temperaturen bis zu 1100°C durchgeführt wird.

9. Verfahren nach einem oder mehreren der Ansprüche 1 bis 8, dadurch gekennzeichnet, dass der Glaskohlenstoff aktiviert wird.

10. Verwendung des nach dem Verfahren nach einem oder mehreren der Ansprüche 1 bis 9 hergestellten Glaskohlenstoffs als Elektrodenmaterial für elektrochemische Doppelschichtkondensatoren.

## Claims

1. Process for the production of vitreous carbon by hardening and pyrolysis of thermosetting carbonisable resins, characterised in that, for the production of porous vitreous carbon, 1 to 10 mass % of a residue-free sublimable salt of a fatty acid with 10 to 20 carbon atoms, which is soluble in the resin, are admixed with the resin.

2. Process according to claim 1, characterised in that furfuryl alcohol is used as resin.

3. Process according to claim 1, characterised in that a phenol-furan resin is used.

4. Process according to one of claims 1 to 3, characterised in that zinc stearate is admixed with the resin.

5. Process according to one of claims 1 to 3, characterised in that zinc undecylenate is admixed with the resin.

6. Process according to one or several of claims 1 to 5, characterised in that p-toluene sulphonic acid is used for hardening of the resin.

7. Process according to one or several of claims 1 to 6, characterised in that the resin is hardened at temperatures of 150 to 200°C.

8. Process according to one or several of claims 1 to 7, characterised in that the pyrolysis is carried out at temperatures up to 1100°C.

9. Process according to one or several of claims 1 to 8, characterised in that the vitreous carbon is activated.

10. Use of the vitreous carbon produced by the process according to one or several of claims 1 to 9 as electrode material for electrochemical double layer capacitors.

**Revendications**

1. Procédé de préparation de carbone vitreux par durcissement et pyrolyse de résines thermodurcissables et susceptibles d'être carbonisées, caractérisé en ce qu'il consiste à ajouter à la résine, pour préparer du carbone vitreux poreux, de 1 à 10% en poids d'un sel, soluble dans la résine et sublimable sans résidus, d'un acide gras ayant de 10 à 20 atomes de carbone.

2. Procédé suivant la revendication 1, caractérisé en ce qu'il consiste à utiliser de l'alcool furfurylique comme résine.

3. Procédé suivant la revendication 1, caractérisé en ce qu'il consiste à utiliser une résine phénolfuranique.

4. Procédé suivant l'une des revendications 1 à 3, caractérisé en ce qu'il consiste à ajouter du stéarate de zinc à la résine.

5. Procédé suivant l'une des revendications 1 à 3, caractérisé en ce qu'il consiste à ajouter de l'undécylénate de zinc à la résine.

6. Procédé suivant l'une ou plusieurs des revendications 1 à 5, caractérisé en ce qu'il consiste à utiliser de l'acide p-toluènesulfonique pour durcir la résine.

7. Procédé suivant l'une ou plusieurs des revendications 1 à 6, caractérisé en ce qu'il consiste à durcir la résine à des températures de 150 à 200°C.

8. Procédé suivant l'une ou plusieurs des revendications 1 à 7, caractérisé en ce qu'il consiste à effectuer la pyrolyse à des températures allant jusqu'à 1100°C.

9. Procédé suivant l'une ou plusieurs des revendications 1 à 8, caractérisé en ce qu'il consiste à activer le carbone vitreux.

10. Utilisation du carbone vitreux préparé par le procédé suivant l'une ou plusieurs des revendications 1 à 9, comme matériau d'électrodes pour des condensateurs électrochimiques à double couche.